(19) Europäisches Patentamt
European Patent Office
Office européen des brevets

(11) **EP 4 769 430 A1**

(12) **EUROPEAN PATENT APPLICATION**

(43) Date of publication:
**01.07.2026 Bulletin 2026/27**

(21) Application number: **25225573.2**

(22) Date of filing: **19.12.2025**

(51) International Patent Classification (IPC):
**G16H 50/30** (2018.01)   **G16H 10/20** (2018.01)
**G16H 50/20** (2018.01)

(52) Cooperative Patent Classification (CPC):
**G16H 50/30; G16H 10/20; G16H 50/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **27.12.2024 IN 202421103823**

(71) Applicant: **Tata Consultancy Services Limited Maharashtra (IN)**

(72) Inventors:
• **MOHAPATRA, Anwesha**
**411028 Pune, Maharashtra (IN)**
• **PAUL, Angela**
**411057 Pune, Maharashtra (IN)**
• **HAQUE, Mohammed Monzoorul**
**411028 Pune, Maharashtra (IN)**
• **DUTTA, Anirban**
**411057 Pune, Maharashtra (IN)**
• **SUKLA RUPANAGUNTLA, Krishna Kishore**
**411057 Pune, Maharashtra (IN)**

(74) Representative: **Goddar, Heinz J.**
**Boehmert & Boehmert**
**Anwaltspartnerschaft mbB**
**Pettenkoferstrasse 22**
**80336 München (DE)**

(54) **METHOD AND SYSTEM FOR PREDICTING PREGNANCY COMPLICATIONS USING A RANKED INCREMENTAL CUMULATIVE RISK (RICR) SCORING**

(57)     This disclosure relates generally to method and system for predicting pregnancy complications using a ranked incremental cumulative risk (RICR) scoring. During pregnancy, undetected medical or obstetric events may result in complications that pose risks to both the mother and the neonate. The method displays a questionnaire comprising a plurality of predefined questions on a clinical device to be answered by a subject. In response from the subject a plurality of answers and one or more unanswered questions for the questionnaire are received to compute a plurality of risk score. Then, a ranked incremental cumulative risk score is computed for the plurality of risk scores using a ranked incremental cumulative risk (RICR) scoring technique to generate a rank index. Finally, the risk index, an action plan, a risk tier, and an action plan is displayed on the clinical device to notify a clinician with a machine-generated alert.

FIG. 2

**Description**

CROSS-REFERENCE TO RELATED APPLICATIONS AND PRIORITY

The present application claims priority from Indian application no. 202421103823, filed on December 27, 2024.

TECHNICAL FIELD

The disclosure herein generally relates to obstetric care, and, more particularly, to method and system for predicting pregnancy complications using a ranked incremental cumulative risk (RICR) scoring.

BACKGROUND

Pregnancy represents a transformative and pivotal phase in a woman's life, characterized by the potential for unexpected medical complications that may result in grave consequences, including maternal and infant fatalities. Medical conditions including pre-eclampsia, postpartum hemorrhage, and premature delivery constitute major factors contributing to these unfavorable outcomes. On a worldwide scale, maternal death rates continue to be elevated, particularly within geographic regions encompassing Africa, South Asia, Latin America, and the Middle East. In nations such as India, a considerable percentage of pregnancies are designated as high-risk, thereby substantially contributing to perinatal morbidity and mortality statistics.

Throughout the gestational period, unidentified medical or obstetric complications may culminate in conditions that endanger both maternal and neonatal well-being. Therefore, consistent prenatal medical supervision are essential. The inability to identify crucial prompt recognition of various gestational complications may manifest through symptoms including a chronic pelvic discomfort, a vaginal hemorrhaging, a pre-eclampsia, and a postpartum bleeding. Failure to identify and manage these risks appropriately can result in maternal and neonatal mortality. This underscores the critical importance of regular antenatal care preventive measures, and early detection of potential complications. Effective management of pregnancy-related risks, therefore, requires timely detection, prompt intervention, treatment of symptoms, consistent antenatal checkups, and professional care during labor and the postpartum period. Early and accurate identification of risks is essential for improving maternal and neonatal health outcomes and reducing mortality rates.

Conventional methods of risk assessment use objective criteria to facilitate early detection and intervention, addressing potential complications. These methods typically categorize the pregnant women into different risk groups. Existing antenatal scoring methods assign weights to individual risk factors and computing a risk score, which, upon surpassing a threshold, indicates whether a pregnant woman is at risk. Although, direct summation approach to risk assessment appears logical, it is not without its limitations. Such methods can yield ambiguous outcomes, resulting in information loss. It is essential to recognize that distinct risk factors are assigned varying weights, and a straightforward summation does not differentiate between individuals who may have identical total scores but possess disparate contributing risk profiles. The significance of the total risk score depends upon the specific combination of risk factors.

In one existing scoring method, antenatal risk factors are considered to compute a fetal risk based on three categories pre-pregnancy data, conditions developing during the current pregnancy (before onset of labor), and gestational age at the time of scoring. The risk to the fetus is indicated by either a low or high value. Based on the identified risk management techniques before, during, and after labor are adjusted. However, existing methods lack the ability to score intermediate risk factors or provide personalized care strategies tailored to individual risk profiles, which could enhance maternal and perinatal outcomes through more effective management and proactive healthcare practices. Currently, no effective methods exists to predict these pregnancy complications early by assessing all risks with appropriate similarity scoring. By the time symptoms of these conditions appear, it is often too late for effective clinical intervention.

SUMMARY

Embodiments of the present disclosure present technological improvements as solutions to one or more of the above-mentioned technical problems recognized by the inventors in conventional systems. For example, in one embodiment, a system to predict pregnancy complications using a ranked incremental cumulative risk (RICR) scoring is provided. The system includes to display electronically on a clinical device, a questionnaire comprising a plurality of predefined questions to be answered by a subject, and in response receiving from the subject a plurality of answers for at least a subset of the plurality of predefined questions in the questionnaire. Each question is mapped to one or more risk factors.

Further, a plurality of risk scores are computed using (i) the plurality of answers received from the subject for each of the plurality of predefined questions in the questionnaire, and (ii) one or more unanswered questions, wherein a default risk score is assigned to each unanswered question.

Then, a ranked incremental cumulative risk (RICR) scoring technique computes a ranked incremental cumulative risk score using the plurality of risk scores, a plurality of proportionate scores, and a proportionate addition score.

Finally, a risk index of the subject is generated using the ranked incremental cumulative risk score to predict one or more pregnancy complications, and displaying the risk index, an action plan and a risk tier on the clinical device to notify a clinician with a machine-generated alert. The risk tier indicates one or more mitigations of each risk event occurring with one or more pregnancy complications at different risk levels. The risk index summarizes an overall probability of the risk event. The action plan is recommended based on the risk index, and the risk tier.

In another aspect, a method to predict pregnancy complications using a ranked incremental cumulative risk (RICR) scoring is provided. The method includes to display electronically on a clinical device, a questionnaire comprising a plurality of predefined questions to be answered by a subject, and in response receiving from the subject a plurality of answers for at least a subset of the plurality of predefined questions in the questionnaire. Each question is mapped to one or more risk factors.

Further, a plurality of risk scores are computed using (i) the plurality of answers received from the subject for each of the plurality of predefined questions in the questionnaire, and (ii) one or more unanswered questions, wherein a default risk score is assigned to each unanswered question.

Then, a ranked incremental cumulative risk (RICR) scoring technique computes a ranked incremental cumulative risk score using the plurality of risk scores, a plurality of proportionate scores, and a proportionate addition score.

Finally, a risk index of the subject is generated using the ranked incremental cumulative risk score to predict one or more pregnancy complications, and displaying the risk index, an action plan and a risk tier on the clinical device to notify a clinician with a machine-generated alert. The risk tier indicates one or more mitigations of each risk event occurring with one or more pregnancy complications at different risk levels. The risk index summarizes an overall probability of the risk event. The action plan is recommended based on the risk index, and the risk tier.

In yet another aspect, there are provided one or more non-transitory machine-readable information storage mediums comprising one or more instructions which when executed by one or more hardware processors cause: displaying electronically on a clinical device, a questionnaire comprising a plurality of predefined questions to be answered by a subject, and in response receiving from the subject a plurality of answers for at least a subset of the plurality of predefined questions in the questionnaire. Each question is mapped to one or more risk factors.

Further, a plurality of risk scores are computed using (i) the plurality of answers received from the subject for each of the plurality of predefined questions in the questionnaire, and (ii) one or more unanswered questions, wherein a default risk score is assigned to each unanswered question.

Then, a ranked incremental cumulative risk (RICR) scoring technique computes a ranked incremental cumulative risk score using the plurality of risk scores, a plurality of proportionate scores, and a proportionate addition score.

Finally, a risk index of the subject is generated using the ranked incremental cumulative risk score to predict one or more pregnancy complications, and displaying the risk index, an action plan and a risk tier on the clinical device to notify a clinician with a machine-generated alert. The risk tier indicates one or more mitigations of each risk event occurring with one or more pregnancy complications at different risk levels. The risk index summarizes an overall probability of the risk event. The action plan is recommended based on the risk index, and the risk tier.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG.1 illustrates an exemplary system to predict one or more pregnancy complications using a ranked incremental cumulative risk (RICR) scoring, according to some embodiments of the present disclosure.

FIG.2 depicts a process flow of the system for predicting the one or more pregnancy complications using the system of FIG.1, in accordance with some embodiments of the present disclosure.

FIG.3 illustrates an incremental proportionate addition score generated for predicting the one or more pregnancy complications using the system of FIG.1, in accordance with some embodiments of the present disclosure.

FIG.4 depicts a graphical representation of the contributions of example individual weights of an antenatal risk score using the system of FIG.1, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

**GLOSSARY:**

As used herein the term "**pregnancy related risks**" or "pregnancy complications" refers to obstetric complications that may occur during pregnancy, childbirth, or postpartum period, and which are typically identified through clinical risk assessments. These pregnancy related risks include but are not limited to conditions such as a pre-eclampsia, a gestational diabetes, a preterm labor, a postpartum hemorrhage, and a fetal abnormalities. Pregnancy related risks are typically associated with a combination of maternal and/or fetal risk factors that can potentially result in adverse health outcomes for both the mother and the neonate if not properly managed or addressed.

As used herein, the term "**Risk factor**" is referred as specific factors, condition or characteristic considered for computing an antenatal risk scoring that may increase likelihood of the one or more pregnancy complications occurring in individual subject. Examples of risk factor may include maternal health conditions (e.g., hypertension, diabetes), obstetric history (e.g., previous preterm birth, prior pregnancy complications), age, lifestyle factors (e.g., smoking, nutrition), or other clinically identifiable indicators. In the context of antenatal risk scoring, risk factor is considered one of the individual elements assessed and used to determine the overall risk level for a pregnant individual.

The term **"Weights"** are referred as assigned numerical values indicating relative importance or severity of each risk factor. In other words, the term "weights" refers to numerical values assigned to each risk factor in an antenatal risk scoring model. These weights represent relative importance or severity of a particular risk factor that contributes to likelihood of pregnancy complications. The weight assigned to each risk factor is based on its association with adverse pregnancy outcomes with higher weights indicating greater significance in overall risk profile. Weights are used to quantitatively differentiate between relative contributions of different risk factors in the overall risk assessment.

As used herein, the term **"Total antenatal risk score"** refers to a numerical score obtained because of the assessment, by summing the individual weight values of all applicable risk factors identified for a given subject. This score represents an overall risk profile of the individual based on the presence of various risk factors, indicating a higher likelihood of experiencing a pregnancy-related complication. The total risk score is used to categorize individuals into specific risk groups (e.g., low, moderate, high, or extremely high risk) and to guide clinical decision-making regarding the necessary interventions or preventive measures to mitigate the identified risks.

As used herein, the term **"Proportionate addition"** refers to the individual value added to the total risk score occurring across a plurality of responses and dependent on the weight of the risk factor and the coefficient as per the type of response.

As used herein, the term **"Cumulative proportionate addition"** refers to the individual value added to the total risk score occurring across a plurality of responses and dependent on the weight of the risk factor and the coefficient as per the type of response.

As used herein, the term "Ranked Incremental Cumulative Risk Scoring" or **RICR,** also referred to as **Ranked Incremental Risk or Ranked Proportionate Addition,** refers to the antenatal risk scoring methodology introduced in this invention. RICR is a computational technique that: (a) sorts per-question weighted scores in descending order, (b) applies an incremental proportionate addition rule to combine these scores, (c) produces a bounded, monotonic cumulative risk index that reflects the relative contribution of each risk factor. The **RICR score** (or ranked incremental cumulative risk score) is the antenatal risk index generated by this method and is used to stratify subjects into risk tiers for clinical decision support. Unlike traditional additive scoring, RICR accounts for unanswered questions, resolves ties deterministically, and mitigates overestimation caused by correlated factors.

The term **"Previous Cesarean Section (PCS)"** refers to the subject or pregnant women who has undergone at least one C-section in a prior pregnancy.

As used herein, the term **"Previous Gynecological Surgery (PGS)"** refers to Previous gynecological surgery refers to any operation performed on the female reproductive organs, including procedures for fibroids, endometriosis, ovarian cysts, and cancer.

As used herein, the term **"Present Pregnancy Blood Pressure (PPBP)"** refers to blood pressure reading during the current pregnancy.

As used herein, the term **"Undernutrition (UN)"** refers to present pregnancy conditions which is a form of malnutrition where the body doesn't receive enough energy and nutrients to maintain health, often leading to conditions like wasting (low weight for height), stunting (low height for age), and underweight.

As used herein, the term **"Other Disorders (OD)"** refers to pre-existing conditions or dental issues and the like.

As used herein, the term **"Pregnancy-Induced Hypertension (PIH)"** refers to a type of high blood pressure that develops after 20 weeks of pregnancy in a woman who previously had normal blood pressure.

As used herein, the term **"Still Birth or Neonate Issues (SN)"** refers to past obstetrical history, the death of a baby before or during birth, while a neonatal death is the death of a baby after birth within the first 28 days of life.

As used herein, the term **"Action plan"** refers to one or more suggested mitigations, options, or recommendations generated for clinician review. The action plan does not constitute a mandatory clinical directive or diagnosis but rather provides possible courses of action or mitigations for consideration by a qualified healthcare professional.

Pregnancy is a period of constant physiological change, during which any previously unobserved medical or obstetric event can lead to complications for both the mother and the neonate. Managing pregnancy risks requires timely detection and intervention for the treatment of symptoms, regular antepartum checkups, and professional care during labor and the postpartum period. Existing scoring schemes have certain limitations such as "antenatal risk assessment" which predominantly relies on an establishment of fixed thresholds for risk level categorization. This approach does not adequately account for complex interrelationships between various risk factors thereby limiting the capacity to discern varying degrees of risk contribution. Further, traditional scoring methods often use simplistic summation methods that aggregate individual risk factors into a total score. However, this method lacks the necessary granularity to evaluate the gravity and specific impact of each contributing risk factor on the overall risk assessment which may often lead to significant errors in risk stratification. Then, the distinction between questionnaires that have not been completed and those that have been answered negatively is not considered. This limitation undermines the accuracy of risk evaluations, as it fails to adequately distinguish between incomplete data and confirmed absence of risk factors.

Traditional scoring systems often rely on a simplistic summation approach that aggregates individual risk factors into a total score. However, as described above, these approaches lack the granularity to evaluate the gravity and specific impact of each contributing risk factor on overall risk assessment, which may result in imprecise risk stratification. Existing antenatal risk assessment methods rely on the establishment of fixed thresholds for categorizing risk levels. These methods fail to account for the complex interrelationships between various risk factors, thus limiting their ability to discern varying degrees of risk contribution.

Moreover, the distinction between questions that have not been answered and those that have been answered negatively undermines the accuracy of risk evaluations. As a result, this may hinder the formulation of recommendations and interventions that are tailored to the specific needs identified for each individual.

Embodiments of the present disclosure provides a method and system to predict pregnancy complications using a ranked incremental cumulative risk (RICR) scoring. The method pertains to obstetric care focusing on evaluation and management of pregnancy related risks to mitigate complications for both the mother (or subject) and the neonate. This systematic approach enables the healthcare providers to tailor interventions and manage pregnancies effectively. In this method, the subject provides a plurality of answers for a plurality of predefined questions in response to a questionnaire displayed on an electronic device (or provided in form of a manual questionnaire to assess the health condition. The plurality of answers are processed to generate a risk index which provides valuable insights to a clinician or the healthcare provider, helping them stratify the subject into appropriate risk categories. This aids in strategizing management methods for such pregnancy complications more effectively. The risk index also facilitates creation of personalized care plans aimed at improving outcomes for both subject or mothers and the newborn, ensuring optimal health and well-being throughout the pregnancy journey. Here, the subject may be a pregnant mother, caregiver or the like.

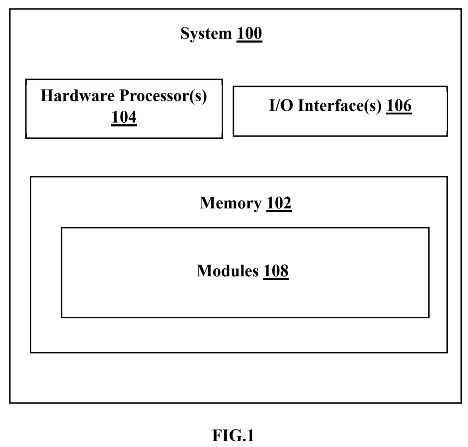

The ranked incremental cumulative risk (RICR) score provides an accurate risk assessment of factors that, when considered individually, may not be deemed high-risk, but when taken combined indicates a potential for complications to arise. The collective accumulation of such risk factors may point to a potential risk, which can be mitigated through appropriate interventions, if identified early. Additionally, the present disclosure categorizes the pregnant women (or subjects) into one or more risk groups enabling personalized care strategies tailored to individual risk profiles. This enhances maternal and perinatal outcomes through effective management and proactive healthcare practices. Additionally, the method also addresses the inadequacies of existing scoring schemes that rely on straightforward summation, failing to account for scenarios in which subjects either lack information or confirm the absence of specific risk factors. The disclosed system is further explained in the method described in conjunction with FIG.1 to FIG.4 below.

Referring now to the drawings, and more particularly to FIG.1 through FIG.4, where similar reference characters denote corresponding features consistently throughout the figures, there are shown preferred embodiments, and these embodiments are described in the context of the following exemplary system and/or method.

FIG.1 illustrates an exemplary system to predict one or more pregnancy complications using a ranked incremental cumulative risk (RICR) scoring, according to some embodiments of the present disclosure. In an embodiment, the system **100** includes processor (s) **104,** communication interface (s), alternatively referred as or input/output (I/O) interface(s) **106,** and one or more data storage devices or memory **102** operatively coupled to the processor (s) **104.** The system **100,** with the processor(s) is configured to execute functions of one or more functional blocks of the system **100.**

The I/O interface(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface (s) 106 can include one or more ports for connecting a number of devices (nodes) of the system 100 to one another or to another server.

The memory 102 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes.

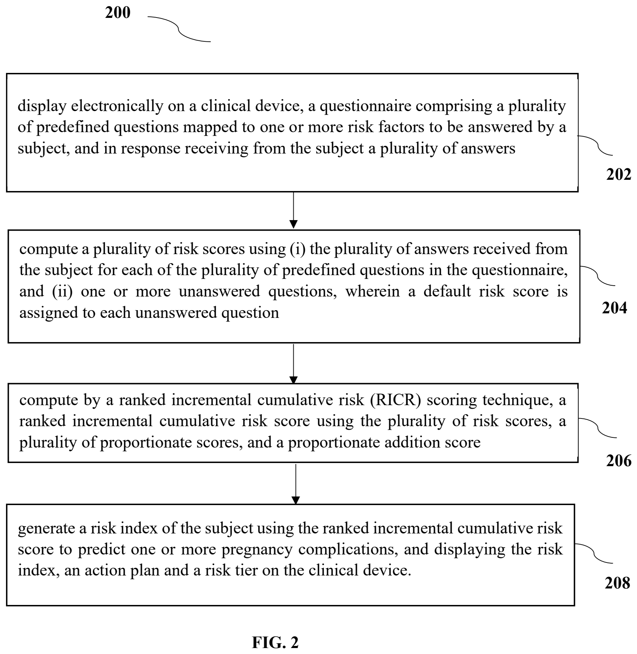

In an embodiment, the memory 102 includes a plurality of modules 108 and can also include various sub-modules as depicted in FIG.2. The plurality of modules 108 include programs or coded instructions that supplement applications or functions performed by the system 100 for executing different steps involved in the process of providing data privacy in service operations of the system 100. The plurality of modules 108, amongst other things, can include routines, programs, objects, components, and data structures, which perform particular tasks or implement particular abstract data types. The plurality of modules 108 may also be used as, signal processor(s), node machine(s), logic circuitries, and/or any other device or component that manipulates signals based on operational instructions. Further, the plurality of modules 108 can be used by hardware, by computer-readable instructions executed by the one or more hardware processors 104, or by a combination thereof.

The memory 102 may comprise information pertaining to input(s)/output(s) of each step performed by the processor(s) 104 of the system 100 and methods of the present disclosure. Functions of the components of system 100, for evaluating pregnancy complications using the RICR scoring, are explained in conjunction with FIG.2 providing flow diagram, architectural overviews, and performance analysis of the system 100.

As an illustrative example scenario for the purpose of explanation the system 100 considers two subjects (e.g., a first pregnant woman or caregiver, and a second pregnant woman or caregiver) referred to as a first subject and a second subject with risk factors assigned weights based on the Das and Dutta scoring method, as indicated in Table 1 below.

The present disclosure addresses limitations of conventional risk scoring schemes to assess severity of individual risk factors as illustrated in Table 1 below. For instance, when two subjects are compared as subject A and subject B using a Das and Dutta scoring scheme (as shown in Table 1), and the total risk per subject is calculated by simple summation, both may yield the same total score of 8. However, the subject A's responses in the affirmative are primarily for high-weighted physiological risks such as hypertension, and diabetes, while subject B's responses predominantly involve relatively lower-weighted risks associated with advanced maternal age and infertility. This disparity in risk factor severity is crucial but obscured by simple summation methods which fail to differentiate between varying levels of risk complexity. Furthermore, simple summation of the risk weights might lead to over estimation of risk score. Therefore, the present disclosure seeks to provide a more nuanced assessment that accurately reflects the relative risk posed by different combinations of risk factors, ensuring appropriate and tailored management strategies based on comprehensive risk profiles.

Table 1 - Example risk factors, assigned weights as per Das & Dutta Scoring scheme, and resulting risk scores of two subjects

| Risk Factors | Weights | First subject 'A' | Second subject 'B' |
|---|---|---|---|
| Age between 16-35 | 0 | Y | N |
| Age > 35 | 2 | N | Y |
| Parity 0 | 2 | Y | N |
| Parity 5 and above | 2 | N | Y |
| Infertility | 1 | N | Y |
| Abortion | 1 | N | Y |
| Bleeding < 20 weeks | 1 | N | Y |
| Previous gynecological surgery | 1 | Y | N |
| Hypertension | 2 | Y | N |
| Diabetes | 3 | Y | N |
| Pregnancy induced hypertension | 1 | N | Y |
| | | | |
| **Total Risk Score** | | **8** | **8** |

Table 1 captures a profile of one or more risk factors with corresponding assessments for the subjects A and B. Although both the subjects exhibit an identical risk score (8 points), their risk profiles show discrepancies. The first subject A might be at a higher risk due to many underlying physiological conditions, whereas the second subject B risks can be attributed to advanced maternal age, abortion, and infertility issues. Here, the one or more risks depends on varied impact of the individual risk factor and a simple summation of scores these risk does not elucidate which subject faces a greater overall risk relative to the other. However, such inadequacies of existing scoring schemes, which rely on straightforward summation, fail to account for scenarios where patients either lack information or confirm the absence of specific risk factors. Additionally, existing scoring schemes fail to consider unanswered questions or ignored questions from the subject.

Furthermore, a mere summation of risk scores does not elucidate which subject faces a greater overall risk relative to the other. Taking these aspects into consideration, the analysis results "should address whether both the subjects receive identical treatment" or "whether tailored management strategies" should be applied based on their distinct risk profiles. This addresses the necessity of refining risk scoring methods to ensure more precise risk scoring and assessment and corresponding management in antenatal care or therapy.

Another inadequacy in existing scoring schemes, which may lead to ambiguous results is illustrated in Table 2. In addition, the Table 2 compares responses from the subject A and the subject B to various risk factors indicating whether the risk factor was not applicable (NA), absent (N), or not provided (NA). Each risk factor is assigned with a specific weight based on its potential impact on pregnancy outcomes.

Table 2 - Risk Factors and Subjects Responses

| RISK FACTOR | WEIGHTS | subject A | subject B |
|---|---|---|---|
| Oedema | 3 | NA | N |
| Chronic renal disease | 3 | NA | N |
| Anemia | 2 | N | NA |

For instance, as illustrated in Table 2, the subject A and the subject B may have unanswered or negatively answered questions regarding certain risk factors such as Oedema, Chronic Renal Disease, and Anaemia. Current methodologies often treat such cases equally, potentially overlooking underlying risks that may become evident through further diagnostic testing. The present disclosed method incorporates a RICR scoring technique that distinguishes between the unanswered and negatively answered questions. This ensures a more accurate assessment by applying a penalty factor of 0.1 to the weight of the risk factor associated with the unanswered questions acknowledging that uncertainty does not negate the potential presence or impact of a risk.

The present invention contemplates several methods for deriving or generating a default risk score for the one or more unanswered questions in the questionnaire, ensuring that the absence of data does not hinder the risk assessment process. In one embodiment, the default risk score may be derived from population averages, where the score is set based on the typical risk factor values observed in a representative patient population. This approach ensures that the one or more unanswered questions are assigned with the default risk score reflective of general trends, based on the assumption that the patient may fall within the average range for similar individuals.

In another embodiment, the default risk score may be based on historical data, where previous patient responses and outcomes are analyzed to identify common risk scores associated with the one or more unanswered questions. This historical data may be sourced from clinical studies, patient records, or large medical databases, providing a data-driven basis for setting the default. Additionally, the default score may be generated in accordance with a predefined clinical guidelines or expert recommendations, which specify standard risk scores for certain conditions in the absence of specific patient responses. These clinical guidelines may come from authoritative medical bodies or established clinical practices. In some embodiments, the default risk score may be dynamically adjusted based on real-time data or trends observed in the population, such as emerging health risks or shifts in epidemiological data. Finally, the method may offer customization options where the default risk score can be tailored based on individual clinical judgment, patient demographics, or institutional preferences, allowing the healthcare providers to fine-tune the default risk score as necessary for specific patient contexts.

FIG.2 depicts process flow of the system to predict pregnancy complications using the system of FIG.1, in accordance with some embodiments of the present disclosure. In an embodiment, the system **100** comprises one or more data storage devices or the memory **102** operatively coupled to the processor(s) **104** and is configured to store instructions for execution of steps of the method **200** by the processor(s) or one or more hardware processors 104. The steps of the method 200 of the present disclosure will now be explained with reference to the components or blocks of the system **100** as depicted in FIG.2 through FIG.4. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

Referring now to the steps of the method **200,** at step **202** the one or more hardware processors **104** displays electronically on a clinical device, a questionnaire comprising a plurality of predefined questions to be answered by a subject, and in response receiving from the subject a plurality of answers for at least a subset of the plurality of predefined questions in the questionnaire, wherein each question is mapped to one or more risk factors.

Examples of the clinical devices includes, but are not limited to, desktop computers, laptops, tablets, and smartphones used by clinicians in hospital wards, clinics, or remote healthcare settings. Additionally, the clinical device may include specialized medical devices integrated with electronic health record (EHR) systems or clinical decision support systems (CDSS) that allow for seamless interaction with patient data. These devices provide necessary interfaces for the clinicians to access real-time patient information, view recommendations, and make informed decisions based on the antenatal risk scores and associated action plans. The context of the present invention, "clinical device" refers to any electronic or computerized equipment used by a healthcare professional to assist in patient care, diagnosis, and management. The clinical device can receive, displaying, and processing patient data, including the results of risk assessments and action plans generated by the system.

The subject may be a pregnant woman, a caregiver, or the like. The caregiver may also answer the questionnaire on behalf of the pregnant woman, in coordination with the pregnant woman, and serve as the responsible party for the care of the pregnant woman..

Each question from the plurality of predefined questions is mapped to the one or more risk factors empirically through a statistical model, one or more clinical trials, and one or more expert opinions, based on a predefined medical knowledge.

Referring to the above illustrated example as depicted in Table 2, initially each subject provides answers suitable for each questionnaire. The questionnaire includes one or more risk factors tagged to each question depicted with specific illustrations but not limited to dynamic update of questionnaire based on facilitations. Recalling the illustrated use case example which particularly addresses scenarios involving multiple interacting risk factors influencing biological outcomes thereby potentially enhancing efficacy.

As described in Table 2, few risk factors includes a Oedema, a Chronic renal disease but are not limited to an anemia, a diabetes and the like. Various risk factors indicates whether the risk factor may or may not be applicable uniformly for each subject or not provided. Each risk factor is assigned to a specific weight based on its potential impact on pregnancy outcomes. For instance, it is assumed that the first subject or the second subject may have unanswered or negatively answered questions or positively answered questions regarding certain risk factors such as oedema, chronic renal disease, previous gynecological surgery, hypertension and anemia and thereof.

Notably, each risk factor is associated with a risk weightage and a coefficient value where each risk factor indicates medical or clinical observations empirically mapped to potential pregnancy related complications.

Referring to Table 3, the questionnaire may include (for example number of questions= 8) from the list of risk factors. Of these in case of subject A, five questions each may have been answered in affirmative, one question negatively answered and two have not been answered. (**YES=5, NO=1, NA=2**). It is noted that unanswered question is also considered in the present disclosure to compute risk score.

Table 3 - Example questionnaire answered by each subject

| First subject | | | | Second subject | | |
|---|---|---|---|---|---|---|
| **Risk factor** | **Weight** | **response** | | **Risk factor** | **weight** | **response** |
| Previous gynecological surgery | 2 | Y | | Age > 35 | 2 | Y |
| Diabetes | 3 | Y | | Parity 5 and above | 2 | Y |
| Age between 16-35 | 0 | Y | | Infertility | 1 | Y |
| Hypertension | 2 | Y | | Abortion | 1 | Y |
| Parity 0 | 2 | Y | | Bleeding < 20 weeks | 1 | Y |
| Oedema | 3 | NA | | Oedema | 3 | N |
| Chronic renal disease | 3 | NA | | Chronic renal disease | 3 | N |
| Anemia | 2 | N | | Anemia | 2 | NA |
| | | | | Pregnancy induced hypertension | 1 | Y |

(continued)

| First subject | | | | Second subject | | | |
|---|---|---|---|---|---|---|---|
| **Risk factor** | | **Weight** | **response** | **Risk factor** | | **weight** | **response** |
| Simple Cumulation | 8 | RICR Method | 5.884 | Simple Cumulation | 8 | RICR Method | 5.48 |

At step **204** of the method **200,** the one or more hardware processors **104** is configured to compute a plurality of risk scores using (i) the plurality of answers received from the subject for each of the plurality of predefined questions in the questionnaire, and (ii) one or more unanswered questions, wherein a default risk score is assigned to each unanswered question.

The plurality of answers are received as response from the subject for the questionnaire. To compute the plurality of risk scores, initially weights are initialized assigned to each question, $w_i$ and coefficient values, $c_i$ corresponding to the response or answer is provided (***yes: +1, no: 0, unanswered: 0.1***). Further, the plurality of risk scores are computed based on a product of a coefficient value (based on the type of response) of each predefined question with a corresponding weight. Here, each risk factor is associated with a risk weightage and the coefficient value.

Each risk factor indicates medical or clinical observations empirically mapped to one or more potential pregnancy related complications.

The coefficient value includes a previous value of the question answered by the subject, a current value of the question answered by the subject, and a next value for the question unanswered by the subject.

Here for the values of Table 3, for *each question* $q_i$ risk score $w_i c_i$ is computed using an equation 1, For example, for the first subject A. the risk factor may be "Previous gynecological surgery" and its weight as "2" along with response "Y", and for the second subject the risk factor may be "Age > 35", weight "2" and response "Y". The below mentioned weights may be represented as in the equation 1.

$$where, \; w_i c_i = 1 * (w_i) \;\;, if \; response =' \, Yes'; w_i c_i =$$
$$0, \; if \; response =' \, No'; and$$

$$w_i c_i = 0.1 * (w_i), if \; response =' \, None' \; i.e \; not \; answered, \; and \; where \; w_i =$$
$$RiskScore(q_i) \; \text{--- equation 1}$$

The list of weights for the first subject weights are $w_1$ = [2,3,0,2,2,3,3,2] and the list of corresponding coefficients are $c_1$ = [1,1,1,1,1,0.1,0.1,0], and for the second subject weights $w_2$ = [2,2,1,1,1,3,2,1] and the list of corresponding coefficients are $c_2$ = [1,1,1,1,1,0.1,0.1,0]. For the above Table 3 the first subject A is $w.c$ = [$w_1 c_1$, $w_2 c_2$, $w_3 c_3$, $w_4 c_4$, $w_5 c_5$, $w_6 c_6$, $w_7 c_7$, $w_8 c_8$] = [1,3,0,2,2,0.3,0.3,0]

Now at step **206** of the method **200,** the one or more hardware processors **104** is configured to compute a ranked incremental cumulative risk score using the plurality of risk scores, a plurality of proportionate scores, and a proportionate addition score, by using a ranked incremental cumulative risk (RICR) scoring technique.

Once the plurality of risk scores are computed for the questionnaire from the above step 204, here at step 206a, the risk weightage are initialized corresponding to each of the predefined question associated with the questionnaire. Further at step 206b, the plurality of risk scores are obtained from the above step 204. Then at 206c, a sorted score list $Scores_{list}$ is determined using the plurality of risk scores in descending using an equation 2,

$$Score_{list} = Sort_{desc}(w_1c_1, w_2c_2, w_3c_3, \ldots) \text{ ---- equation 2}$$

$$= [s_1, s_2, s_3, \ldots] \qquad where\ s_1 \geq s_2 \geq s_3 \ldots$$

$$such\ that\ s_1 = \max([w_1c_1, w_2c_2, w_3c_3, \ldots])$$

$$s_2 = \max([w_1c_1, w_2c_2, w_3c_3, \ldots] - [s_1])$$

$$s_3 = \max([w_1c_1, w_2c_2, w_3c_3, \ldots] - [s_1, s_2])$$

$$Scores_{list} = [3,2,2,1,0.3,0.3,0,0,0]$$

Further, at step 206d a rank list is determined by ranking average of the sorted score list corresponding to each risk score using an equation 3 and an equation 4,

$$Rank_{list} = [Rank(s_1), Rank(s_2), Rank(s_3), \ldots] \text{ ---- equation 3}$$

$$Ranks_{list} = [1,2.5,2.5,4,5.5,5.5,7.5,7.5]$$

$$If\ s_i\ is\ unique, Rank(s_i) = \{i\}$$

$$If\ s_i = s_{i+1} = \cdots = s_{i+k-1}, \quad where\ k\ is\ the\ number\ of\ scores\ of\ equal\ value$$

$$Rank(s_i) = Rank(s_{i+1}) = \cdots = Rank(s_{i+k-1})$$

$$= Average\big(Rank(s_i), Rank(s_{i+1}), \ldots, Rank(s_{i+k-1})\big)$$

$$= \frac{Rank(s_i) + Rank(s_{i+1}) + \cdots + Rank(s_{i+k-1})}{k} \text{ ---- equation 4}$$

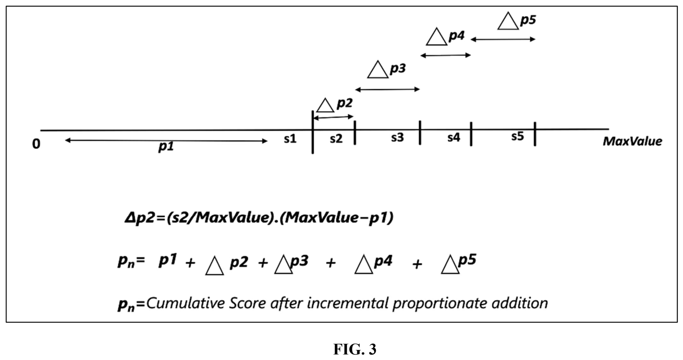

Referring to FIG.3, here from the above step once the sorted score list and the rank list to compute a proportionate addition is performed at step 206e. Here, the proportionate addition is performed when two or more risk scores from a plurality of proportionate scores are highly correlated using the maximum value in the sorted score list and the rank list which are described in later embodiments.

Then, the ranked incremental cumulative risk score is computed using the plurality of risk scores, the plurality of proportionate scores and the proportionate addition score.

When two or more risk factors are highly correlated (linear relationship) then it often becomes difficult to determine the individual effect of each factor by computing the ranked incremental cumulative risk score. The problem of multicollinearity (amongst the plurality of considered risk factors) can be detected using a mathematical tools like Variance Inflation Factor (VIF) and the extent to which the contribution of individual but collinear factors can inflate the overall risk score that can be estimated. Let the maximum of scale *MaxValue* is the sum of all score values in $Scores_{list}$ as in equation 5,

$$MaxValue = \sum_{i=1}^{n} s_i \text{ ---- equation 5}$$

where $s_i \in Scores_{list}$ and n = $|Scores_{list}|$

$$Scores_{list} = 3 + 2 + 2 + 1 + 0.3 + 0.3 + 0 + 0 = 8.6$$

Referring to Table 4, the plurality of proportionate scores are computed by determining a first proportionate score p using a first sorted score from the sorted score list and a first sorted score from the rank list.

Let p represent the ranked incremental cumulative risk score is added through in each iteration. The initial value $p_n$ considers the highest risk score value obtained after sorting $p_n = s_n$. Iteratively performing for each proportionate score by summing the incremental proportionate value for the current term (risk factor at the $i^{th}$ position) as per the rank in the sorted list of factors, to the proportionate score calculated for all the preceding terms. The incremental risk due to the current executing factor in the predefined question is calculated as the product of the contribution of this factor to the MaxValue to that of the remaining risk obtained after subtracting the impact of the previous factors on the risk score with a previous term and a current term $\Delta p_{n+1}$.

Further, each of the proportionate score corresponding to the risk score is determined using (i) a first element from the sorted score list and (ii) a first element from the rank list. Iteratively the proportionate score is computed corresponding to each risk score by summing the proportionate score of a previous term incremental to each risk score of the current term, and sequentially the next term is computed as described in equation 6.

Following this, several iterations are performed across the following values of each risk score in $Scores_{list}$ to obtain $\Delta p_2$ which is incremented due to corresponding individual scores as described below in equation 6,

$$\Delta p_2 = \left(\frac{s_2}{MaxValue}\right).(MaxValue - p_1)$$

$$\text{and, } p_2 = p_1 + \Delta p_2$$

$$\Delta p_3 = \left(\frac{s_3}{MaxValue}\right).(MaxValue - p_2)$$

$$and, p_3 = p_2 + \Delta p_3$$

$$\Delta p_4 = \left(\frac{s_4}{MaxValue}\right).(MaxValue - p_3)$$

$$and, p_4 = p_3 + \Delta p_4$$

$$\text{and ... till obtaining } p_n$$

$$\text{------ equation 6}$$

1. The previous term $\Delta p_{n-1}$ is (i) the sum of a highest risk score, and (ii) the incremental proportionate value for the previous term which is obtained as the product of each risk score of the previous term with respect to the maximum value, and a remaining residual proportion of the maximum value.

2. The current term $\Delta p_n$ is the incremental proportionate value for the previous term which is obtained as the product of each risk score of the current term with respect to the maximum value and the remaining residual proportion of the maximum value.

3. The next term $\Delta p_{n+1}$ is the incremental proportionate value for the current term which is obtained as the product of each risk score of the current term with respect to the maximum value and the remaining residual proportion of the maximum value, and the current term.

Finally, at step 206e, the ranked incremental cumulative risk score is computed using the plurality of proportionate scores as in equation 7,

$$p_i = p_{i-1} + \left(\frac{s_i}{MaxValue}\right).(MaxValue - p_{i-1}) \qquad \forall i \in [2, n]$$

$$FinalScore = p_n \qquad where \; n = |Scores_{list}|$$

$$\text{--- equation 7}$$

Table 4 represents example computed in response for Table 3

Table 4 - Example computation for Table 3

| First Subject |
|---|
| $p_i = p_{i-1} + \left(\frac{s_i}{MaxValue}\right).(MaxValue - p_{i-1}) \qquad \forall i \in [2, n]$ |
| Where MaxValue = 8.6, and ranked score list is $Scores_{list}$ = [3,2,2,1,0.3,0.3,0,0] |

(continued)

| First Subject |
|---|
| Total list of questions answered is 8 including yes=5, no=1 and na=2<br>Ranked incremental cumulative risk score (RICR) = **5.88** |
| **Second Subject** |
| $$p_i = p_{i-1} + \left(\frac{s_i}{MaxValue}\right).(MaxValue - p_{i-1}) \qquad \forall i \in [2, n]$$<br>Where MaxValue = 8.2, and ranked score list is<br>$Scores_{list}$ = [2,2,2,1,1,1,0.2,0,0]<br>Total list of questions answered is 8 including yes=6,<br>no=2 and na=1<br>Ranked incremental cumulative risk score (RICR) = **5.48** |

Finally, at step **208** of the method **200,** the one or more hardware processors **104** is configured to predict a risk index of the subject using the ranked incremental cumulative risk (RICR) score to recommend an action plan, and displaying the risk index, an action plan, and a risk tier on the clinical device to notify a clinician with a machine-generated alert.

The action plan is recommended based on the risk index, and the risk tier. The risk tier indicates one or more mitigations of each risk event occurring with one or more pregnancy complications at different risk levels, and wherein the risk index summarizes an overall probability of the risk event.

"Notifying" in the context of the present invention refers to the process by which the system communicates the generated the risk tier to the clinician, ensuring timely and effective decision-making with the machine-generated alert. This notification can be delivered through various means, including, but not limited to, electronic alerts on clinical devices such as tablets, computers, or smartphones, or through integrated healthcare management software used by the clinician. The notification may take the form of a visual display, such as a pop-up message or dashboard update, which provides the clinician with an overview of the patient's risk score and the corresponding action plan. Additionally, notifications can be sent via secure messaging systems, emails, or mobile notifications to ensure that the clinician is promptly informed. In some embodiments, the system may also include a more detailed report, accessible via a patient management portal or electronic health record (EHR), that allows the clinician to review the full context of the risk assessment and recommended actions. The notification system may be configured to prioritize alerts based on the severity of the risk score, ensuring that the clinician is immediately made aware of high-risk situations requiring urgent attention.

The action plan advises the clinician or the healthcare provider for further testing of the subject, devising a care regimen for the subject that helps avoiding adverse pregnancy complications and pregnancy outcomes.

Also, the action plan may recommend a course of action by comparing the ranked incremental cumulative risk score (RICR) to a predefined threshold or risk levels associated with specific pregnancy complications. These thresholds may be established using clinical guidelines, expert opinions, or historical data, and could include recommendations such as additional testing, medication, or referral to a specialist.

In the context of the present invention, the **"action plan"** refers to a plurality of clinical recommendations or interventions designed to manage, monitor, or mitigate potential pregnancy complications identified through the computed RICR score. The action plan may be a personalized course of action proposed for the subject, tailored to the individual's specific risk factors and clinical condition. Examples of the action plan includes, but are not limited to,

1. recommending additional diagnostic tests (e.g., ultrasound, blood work, or genetic screenings) to further assess pregnancy risks;
2. suggesting a referral to a specialist, such as an obstetrician or maternal-fetal medicine expert, for advanced care;
3. advising the subject on lifestyle modifications (e.g., diet changes, increased physical activity, or smoking cessation) to reduce risk;
4. prescribing preventive medications (e.g., low-dose aspirin for preeclampsia prevention or gestational diabetes management);
5. scheduling more frequent prenatal visits for closer monitoring.

Additionally, the action plan may include emergency protocols or escalation procedures, such as advising immediate hospitalization or interventions if critical thresholds are exceeded in the risk score. The action plan is dynamically generated based on the ranked incremental risk score (RICR) and may consider the subject's unique medical history, preferences, and other relevant clinical factors to ensure the appropriateness and effectiveness of the recommended actions. Also, the clinician can review and select the most appropriate course of the action plan to mitigate the risk event based on the patient's unique medical history, preferences, and other contextual factors. The recommendations and action plans are further tailored to individual patients by incorporating personalized data such as the patient's age, medical conditions, previous pregnancy history, and other relevant factors.

Additionally, the system may utilize machine learning (ML) models to refine its recommendations over time, learning from new patient data and clinical outcomes to improve the accuracy of future suggestions.

The method also considers regional healthcare guidelines, practitioner input, or institutional protocols to ensure that the recommendations are aligned with local practices and standards of care. Furthermore, it allows for customization based on patient-specific needs or the clinician's preferences, ensuring a flexible and dynamic approach to pregnancy care.

As can be observed from the above steps, the ranked incremental cumulative risk score (RICR) which leads to an over estimation of the magnitude of risk faced by both subjects A and B which has been overcome using the ranked cumulation approach. Furthermore, the results obtained from the method demonstrates a notable discrepancy between the risk scores assigned to the subject A and the subject B. In the case of the subject A, not only did a highly weighted risk factor yield a positive response, but also lesser weighted risks and those that remained unanswered. As it has been previously established that the unanswered question may be an indicative of underlying risk, the scores for these factors are included in the total risk score assessment.

In contrast, the RICR for the subject B is comprised of medium and low-level risk factors. Furthermore, the number of unanswered questions is fewer than those provided by the subject A. The methodology employed by the RICR scoring technique facilitates refinement of scores through addition of a refined layer of assessment, enabling the differentiation between cases with similar characteristics. The effect of individual weights on the ranked incremental cumulative risk score and the steps involved for the subject A have been tabulated in Table 5.

### Table 5 – Experimental data of example (Table 3)

| | Risk Factors Included | % of Risk factors included | Score due to risks | Coefficient Values | Product Scores | Sorted Product Scores | RICR Score | % of RICR Score obtained | X | Y |
|---|---|---|---|---|---|---|---|---|---|---|
| | 0 | 0 | 0 | 0 | 0 | 0 | 0 | 0 | 0 | 0 |
| Previous gynecological surgery | 1 | 12.5 | 1 | 1 | 1 | 0 | 0 | 0 | 12.5 | 0 |
| Diabetes | 2 | 25 | 3 | 1 | 3 | 0 | 0 | 0 | 25 | 0 |
| Age between 16-35 | 3 | 37.5 | 0 | 1 | 0 | 0.3 | 0.3 | 3.49 | 37.5 | 3.49 |
| Hypertension | 4 | 50 | 2 | 1 | 2 | 0.3 | 0.6 | 6.98 | 50 | 6.98 |
| Parity 0 | 5 | 62.5 | 2 | 1 | 2 | 1 | 1.6 | 18.6 | 62.5 | 18.6 |
| Oedema | 6 | 75 | 3 | 0.1 | 0.3 | 2 | 3.6 | 41.86 | 75 | 41.86 |

| Chronic renal disease | 7 | 87.5 | 3 | 0.1 | 0.3 | 2 | 5.6 | 65.12 | 87.5 | 65.12 |
|---|---|---|---|---|---|---|---|---|---|---|
| Anemia | 8 | 100 | 2 | 0 | 0 | 3 | 8.6 | 100 | 100 | 100 |

In another embodiment, the ranked incremental cumulative risk score is graphically represented for the plurality of risk scores involved are of varying weights (unequal distribution of weights), making the contribution of each differentially weighted risk factor to the total risk to be varied thus introducing a degree skewness which has been graphically represented. The skewness of the ranked incremental cumulative risk score helps the clinician to recommend the action plan for the subject.

This visual representation of contributing risk factors would be a valuable tool for clinicians or medical professionals attempting to analyze such subject pregnancy complications. The graphical representation of inequality of the contributing factors can be presented in a variety of visualization charts, including pie charts and column charts, among others. In this instance, the contribution of risk factors and the measure of the coefficient have been represented using a Lorenz curve and a Gini coefficient. This is a common method of representing the distribution of wealth or income across a population. The concept has been extrapolated to fit the context of the distribution of risk across the questions answered.

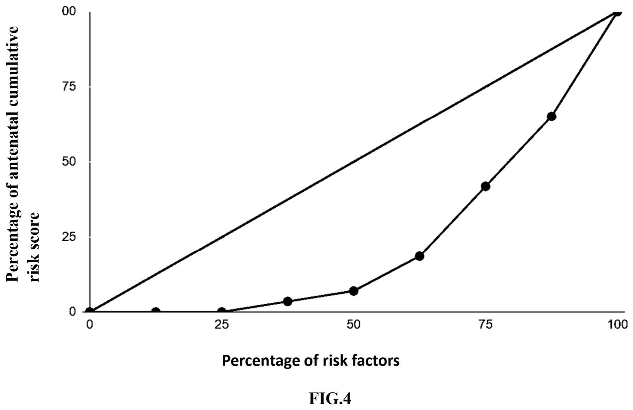

FIG.4 depicts a graphical representation of example individual weights of the RICR score using the system of FIG.1, in accordance with some embodiments of the present disclosure. FIG.4 depicts calculation of the index from the graphical representation of the cumulative scores is done to identify the pattern of distribution of weights across the risk factors. The coefficient value ranges between 0-1 where the values represent perfect equality and maximal inequality respectively. The Gini index (Gi) is defined as the ratio of the area between the perfect equality line and the Lorenz curve (A) divided by the total area under the perfect equality line (A + B). An example illustration of Gini index has been given below. The effect of individual weights on the ranked incremental cumulative risk score for the example case study described above has been plotted in the graph below as per the steps explained.

**Experimental Data with practical examples:** Traditional risk scoring techniques, such as the Das and Dutta method, assign weights to individual risk factors to calculate the cumulative score. However, simple summation can obscure important nuances such as two individuals may receive identical scores despite facing very different clinical realities due to the type and severity of their risks. For example, Table 1 of this specification demonstrates how the Das and Dutta method yielded a risk score of 8 for two subjects, though their underlying risk profiles differed significantly.

Despite global advancements, regions such as South Asia and sub-Saharan Africa face a disproportionate burden of preterm births, exacerbated by limited access to prenatal care and structural inequities. This persistent challenge underscores the need for more refined and context-sensitive approaches to pregnancy risk assessment, particularly in resource-limited settings.

The practical utility of the RICR methodology is predicted using real-world clinical data. For this purpose, the clinical metadata is collected as part of the clinical prospective cohort study, conducted in collaboration with a Lady Hardinge Medical College (LHMC), New Delhi. It is noted that the primary objective of the clinical study was to investigate the role of the vaginal microbiome in preterm birth (PTB), to predict risk scoring methods. However, the rich clinical metadata generated which includes demographic, lifestyle, medical history, and pregnancy outcome data provided an ideal real-world dataset for retrospective evaluation of risk assessment methodologies.

The clinical study was enrolled with 425 pregnant women (all over 18 years of age and in their first trimester) at LHMC, with ethical approval from the LHMC Ethics Committee for Human Research. Written informed consent was obtained from all participants, and the study was conducted in accordance with Good Clinical Practice (GCP) and Indian Council of Medical Research (ICMR) guidelines. Participants were followed through delivery, and comprehensive clinical metadata was systematically recorded.

Referring to Table 6, if the identical score of the subject is 7 in preterm and term subjects, the risk score of adverse pregnancy outcomes are computed as described below in Table 6. The Table 6 depicts comparison between Das & Dutta pregnancy risk factors between preterm and term subjects having identical score of 7 and cumulated using a simple summation vs the RICR.

**Table 6 (Use case example 1):** Comparison of Das & Dutta pregnancy risk factors

| Risk factor weights | PI-101 (Das & Dutta) | PI-101 (RICR) | PI-93 (Das & Dutta) | PI-93 (RICR) |
|---|---|---|---|---|
| Age | 2 | 2 | 0 | 0 |
| Parity | 2 | 2 | 1 | 1 |

(continued)

| Risk factor weights | PI-101 (Das & Dutta) | PI-101 (RICR) | PI-93 (Das & Dutta) | PI-93 (RICR) |
|---|---|---|---|---|
| PCS | 0 | 0.1 | 2 | 2 |
| Abortion | 1 | 1 | 1 | 1 |
| Infertility | 0 | 0 | 0 | 0 |
| PGS | 0 | 0 | 0 | 0 |
| PPBP | 2 | 2 | 2 | 2 |
| UN | 0 | 0.1 | 0 | 0 |
| OD | 0 | 0 | 1 | 1 |
| PIH | 0 | 0.1 | 0 | 0.1 |
| SN | 0 | 0.1 | 0 | 0.1 |
| Cumulative Risk Score | 7 | 5.045 | 7 | 4.868 |
| Pregnancy outcome | Preterm | Preterm | Term | Term |

It is noted from the above table PI-101 (Das and Dutta) and PI-93 (Das and Dutta) refers to anonymized patient identifiers obtained from practical clinical observations.

Additionally, if the identical score of 6 in preterm and term subjects calculated to assess risk of adverse pregnancy outcomes. The below Table 7 represents comparison of Das & Dutta pregnancy risk factors between preterm and term subjects having identical score of 6 and cumulated using a simple summation vs the RICR method.

**Table 7 (Use case example 2)-** Comparison of Das & Dutta pregnancy risk factors

| Risk factor weights | PI-287 (Das & Dutta) | PI-287 (RICR) | PI-115 (Das & Dutta) | PI-115 (RICR) |
|---|---|---|---|---|
| Age | 0 | 0 | 0 | 0 |
| Parity | 3 | 3 | 1 | 1 |
| PCS | 2 | 2 | 2 | 2 |
| Abortion | 1 | 1 | 0 | 0 |
| Infertility | 0 | 0 | 1 | 1 |
| PGS | 0 | 0 | 0 | 0 |
| PPBP | 0 | 0 | 2 | 2 |
| UN | 0 | 0 | 0 | 0 |
| OD | 0 | 0 | 0 | 0 |
| PIH | 0 | 0.1 | 0 | 0.1 |
| SN | 0 | 0.1 | 0 | 0.1 |
| **Cumulative Risk Score** | **6** | **4.44** | **6** | **4.26** |
| **Pregnancy outcome** | **Preterm** | **Preterm** | **Term** | **Term** |

It is noted from Table 7, the PI-287 and PI-115 refer to anonymized patient identifiers obtained from practical clinical observations.

Using the experimental data of Table 6-7, the present method of the invention is experimentally evaluated with below illustrations. The RICR method incrementally weights and aggregates risk factors to resolve ties. Sorting scores ensures the most significant risks are considered first, while proportionate addition reflects their relative contributions.

It is noted, the weights assigned to the particular risk factor are based on presence (yes) - weight corresponding to Das & Dutta factor, absence (no), and unanswered as 0.1.

In Table 7, for the PI score is 287 (Preterm: score tie-6). The proportionate score is computed as,

Step 1: Individual risk scores are [0, 3, 2.0, 1, 0, 0, 0, 0.0, 0, 0.1, 0.1]

Step 2: Sorted scores (descending) are [3.0, 2.0, 1.0, 0.1, 0.1, 0, 0, 0, 0, 0, 0]

Step 3: Max value = 3.0 + 2.0 + 1.0 + 0.1 + 0.1 = 6.2

Step 4: Proportionate Addition as, $p_1 = 3.0$, $p_2 = 3.0 + ((2.0 / 6.2) * (6.2 - 3.0)) = 4.032$, $p_3 = 4.032 + (1.0 / 6.2) * (6.2 - 4.032) = 4.382$, $p_4 = 4.382 + (0.1/6.2) * (6.2 - 4.382) = 4.411$, and $p_5 = 4.411 + (0.1 / 6.2) * (6.2 - 4.411) = 4.440$.

The final RICR Score is 4.440.

In Table 7, if the PI score is 115 (Term: score tie-6),

Step 1: Individual Risk Scores are [0, 1, 2.0, 0, 1, 0, 2, 0.0, 0, 0.1, 0.1]

Step 2: Sorted Scores (descending) are [2.0, 2.0, 1.0, 1.0, 0.1, 0.1, 0, 0, 0, 0, 0]

Step 3: MaxValue = 2.0 + 2.0 + 1.0 + 1.0 + 0.1 + 0.1 = 6.2

Step 4: Proportionate Addition $p_1 = 2.0$, $p_2 = 2.0 + (2.0 / 6.2) \times (6.2 - 2.0) = 3.355$, $p_3 = 3.355 + (1.0 / 6.2) \times (6.2 - 3.355) = 3.814$, $p_4 = 3.814 + (1.0/6.2) \times (6.2 - 3.814) = 4.199$, $p_5 = 4.199 + (0.1 / 6.2) \times (6.2 - 4.199) = 4.231$, and $p_6 = 4.231 + (0.1 / 6.2) \times (6.2 - 4.231) = 4.263$.

The final RICR Score is 4.263

In Table 6, if the PI score is 105 (PreTerm: score tie-7).

Step 1: Individual Risk Scores are [2, 2, 0.1, 1, 0, 0, 2, 0.1, 0, 0.1, 0.1]

Step 2: Sorted Scores (descending) are [2.0, 2.0, 2.0, 1.0, 0.1, 0.1, 0.1, 0.1, 0, 0, 0]

Step 3: MaxValue = 2.0 + 2.0 + 2.0 + 1.0 + 0.1 + 0.1 + 0.1 + 0.1 = 7.4

Step 4: Proportionate Addition are $p_1 = 2.0$, $p_2 = 2.0 + (2.0 / 7.4) \times (7.4 - 2.0) = 3.459$, $p_3 = 3.459 + (2.0 / 7.4) \times (7.4 - 3.459) = 4.524$, $p_4 = 4.524 + (1.0 / 7.4) \times (7.4 - 4.524) = 4.913$, $p_5 = 4.913 + (0.1 / 7.4) \times (7.4 - 4.913) = 4.947$, $p_6 = 4.947 + (0.1 / 7.4) \times (7.4 - 4.947) = 4.980$, $p_7 = 4.980 + (0.1 / 7.4) \times (7.4 - 4.980) = 5.013$, and $p_8 = 5.013 + (0.1 / 7.4) \times (7.4 - 5.013) = 5.045$.

The final RICR score is 5.045

In Table 6, if the PI score as 93 (Term: score tie-7).

Step 1: Individual Risk Scores are [0, 1, 2.0, 1, 0, 0, 2, 0.0, 1, 0.1, 0.1]

Step 2: Sorted Scores (descending) are [2.0, 2.0, 1.0, 1.0, 1.0, 0.1, 0.1, 0, 0, 0, 0]

Step 3: MaxValue = 2.0 + 2.0 + 1.0 + 1.0 + 1.0 + 0.1 + 0.1 = 7.2

Step 4: Proportionate Addition scores are $p_1 = 2.0$, $p_2 = 2.0 + (2.0 / 7.2) \times (7.2 - 2.0) = 3.444$, $p_3 = 3.444 + (1.0 / 7.2) \times (7.2 - 3.444) = 3.966$, $p_4 = 3.966 + (1.0 / 7.2) \times (7.2 - 3.966) = 4.415$, $p_5 = 4.415 + (1.0 / 7.2) \times (7.2 - 4.415) = 4.802$, $p_6 = 4.802 + (0.1 / 7.2) \times (7.2 - 4.802) = 4.835$, and $p_7 = 4.835 + (0.1 / 7.2) \times (7.2 - 4.835) = 4.868$.

The Final RICR score is 4.868.

The comparative analysis of the Das & Dutta vs RICR method provides the clinical metadata acquired in this study enabled a direct comparison between the Das & Dutta scoring method and the Ranked Incremental Cumulative Risk (RICR) methodology introduced in this invention. RICR resolves tied scores and provides greater discriminative power by integrating nuanced weightings and metadata. Illustrations of the use case examples (Table 6 and 7). In Table 6, both a preterm and a term case received a score of 7 using Das & Dutta, but RICR differentiated them (scores: 5.045 vs. 4.868). In Table 7, tie is identified at score 6 was resolved by RICR (scores: 4.44 vs. 4.26).

These instances highlight the limitations of additive scoring and the need for refined methods like RICR. The RICR method effectively disentangles tied scores generated by the Das & Dutta method, revealing meaningful distinctions in pregnancy risk profiles. By integrating nuanced weightings and metadata, RICR introduces resolution where additive scoring falls short. Its discriminative power could be further amplified with richer clinical inputs, highlighting the critical role of comprehensive metadata in enhancing the precision of pregnancy risk prediction. From the viewpoint of clinical significance, this improved discrimination enables more precise risk stratification and potentially better-targeted interventions for high-risk pregnancies.

The present invention contemplates several methods for deriving or generating a default risk score for unanswered questions in the questionnaire, ensuring that the absence of data does not hinder the risk assessment process. In one embodiment, the default score may be derived from population averages, where the score is based on the typical risk factor values observed in a representative patient population. This approach ensures that unanswered questions are assigned a score reflective of general trends, based on the assumption that the patient may fall within the average range for similar individuals. In another embodiment, the default score may be based on historical data, where previous patient responses and outcomes are analyzed to identify common risk scores associated with unanswered questions. This historical data may be sourced from clinical studies, patient records, or large medical databases, providing a data-driven basis for setting the default. Additionally, the default score may be generated in accordance with predefined medical guidelines or expert recommendations, which specify standard risk scores for certain conditions in the absence of specific patient responses. These guidelines may come from authoritative medical bodies or established clinical practices. In some embodiments, the default score may be dynamically adjusted based on real-time data or trends observed in the population, such as emerging health risks or shifts in epidemiological data. Finally, the system may offer customization options where the default score can be tailored based on individual clinical judgment, patient demographics, or institutional preferences, allowing healthcare providers to fine-tune the default scoring model as necessary for specific patient contexts. Through these various methods, the system ensures that the lack of an answer does not detract from the overall accuracy of the risk assessment. In other words, this approach ensures robust handling of missing data: unanswered items receive a non-zero default coefficient, avoiding silent information loss from naive imputation or zeroing.

The present invention contemplates that the model and scoring method may be adaptable and customizable for different patient groups, ensuring applicability across diverse populations. This adaptability can be achieved through machine learning techniques, where the system dynamically updates risk factor mappings based on new patient data, clinical feedback, or emerging medical research. For instance, the model may be fine-tuned for specific demographic groups, such as age, ethnicity, or pre-existing medical conditions, to reflect varying risk profiles. Additionally, the scoring method can incorporate continuous learning from real-time patient data, enhancing its accuracy and relevance over time. In some embodiments, the model may also be configured to account for regional healthcare practices or specific clinical guidelines, enabling personalized recommendations that are tailored to the needs of the individual subject.

The written description describes the subject matter herein to enable any person skilled in the art to make and use the embodiments. The scope of the subject matter embodiments is defined herein and may include other modifications that occur to those skilled in the art. Such other modifications are intended to be within the scope of the present disclosure if they have similar elements that do not differ from the literal language of the present disclosure or if they include equivalent elements with insubstantial differences from the literal language of the embodiments described herein.

The embodiment of present disclosure herein addresses unresolved problem of obstetric care. The embodiment, thus provides method and system to identify pregnancy complications using the ranked incremental cumulative risk scoring. Moreover, the embodiments herein further provide development of RICR scoring method employing ranked incremental increases based on the varying contributions of risk factors, moving beyond simple dichotomous or proportionate values. Refinement of scoring accommodates partially answered questionnaires and differentiates between the severity of contributing risk factors, addressing inherent ambiguities in total scores. Further provides improvement in distinguishing between incomplete questionnaires and negative responses, along with enhanced provision of personalized recommendations tailored to individual risk profiles.

The method provides technical advancements with improved granularity in assessing individual risk factors and enhances differentiation between questionnaire responses to ensure more accurate and tailored management strategies for maternal and neonatal health. The method impedes the precise risk stratification and personalized care planning that is necessary for optimal patient care. The method also provides increased accuracy of risk evaluations and hinders the development of bespoke recommendations and interventions. The necessity for enhanced precision is evident. This enables healthcare providers to gain a deeper understanding of and address the complex interactions that contribute to adverse pregnancy outcomes, stratify the patient as per the risk and the contributing factors and design customized risk management strategies. Additionally, the method of the present disclosure provides 1. numerical stability and boundedness of the cumulative function, 2. Tie-resolution without retraining or clinician heuristics, 3. RICR has improved efficiency with predictable O(n log n) complexity, and handles robust missing data via default coefficient rather than imputation.

The method provides following technical advancements: (a) Bounded, monotonic accumulation: the ranked incremental cumulative rule ensures the computed risk index p remains within [0, S] and increases monotonically as affirmative evidence accrues; (b) Deterministic tie-resolution without retraining: score ties from additive schemes are resolved by ordering and proportionate remainder updates, improving discriminative power for triage; (c) Robust handling of missing data: unanswered items receive a non-zero default coefficient, avoiding silent information loss from naive imputation or zeroing; (d) Stable behavior under multicollinearity: optional detection of correlated factors (e.g., via VIF) prevents over-contribution from collinear features (e) Real-time, low-latency updates on clinical devices: as responses stream in, the risk index updates incrementally without recomputing from scratch, enabling on-device decision support in bandwidth-limited settings; (f) Predictable computational complexity: O(n log n) due to sorting, with linear-time updates thereafter; suitable for resource-constrained clinical endpoints; and (g) Actionable visualization: computation of a concentration metric (e.g., Gini) provides an interpretable view of uneven factor contributions for clinician review.

It is noted that embodiments described herein are discussed in the context of a Large Language Model (LLM) or Artificial Intelligence (AI) model or Machine Learning (ML) model and or with a mentioned training data set. It is to be understood by a person having ordinary skill in the art or person skilled in the art that the referred AI/ML/LLM model(s) are exemplary and shall not be construed as limiting the scope of the present disclosure and they may be trained by any training dataset that meets the mentioned defining characteristics or has characteristics that define the exemplary training dataset mentioned.

**EP 4 769 430 A1**

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method (200) to predict pregnancy complications, the method comprising:

displaying (202) electronically on a clinical device, a questionnaire comprising a plurality of predefined questions to be answered by a subject, via one or more hardware processors, and in response receiving from the subject a plurality of answers for at least a subset of the plurality of predefined questions in the questionnaire, wherein each question is mapped to one or more risk factors;
computing (204) via the one or more hardware processors, a plurality of risk scores using (i) the plurality of answers received from the subject for each of the plurality of predefined questions in the questionnaire, and (ii) one or more unanswered questions, wherein a default risk score is assigned to each unanswered question;
computing (206) by a ranked incremental cumulative risk (RICR) scoring technique via the one or more hardware processors, a ranked incremental cumulative risk score using the plurality of risk scores, a plurality of proportionate scores, and a proportionate addition score; and
generating (208) via the one or more hardware processors, a risk index of the subject using the ranked incremental cumulative risk score to predict one or more pregnancy complications, and displaying the risk index, an action plan and a risk tier on the clinical device to notify a clinician with a machine generated- alert,

18

wherein the risk tier indicates one or more mitigations of each risk event occurring with one or more pregnancy complications at different risk levels,

wherein the risk index summarizes an overall probability of the risk event, and

wherein the action plan is recommended based on the risk index, and the risk tier.

2. The processor implemented method as claimed in claim 1,

wherein each question of the plurality of predefined questions is mapped to the one or more risk factors empirically through a statistical model, one or more clinical trials, and one or more expert opinions, based on a predefined medical knowledge base and

wherein each of the risk factor is associated with a risk weightage and a coefficient value, and wherein each of the risk factor indicates one or more clinical observations empirically mapped to each risk event.

3. The processor implemented method as claimed in claim 2, wherein the coefficient value includes a previous value of the question answered by the subject, a current value of the question answered by the subject, and a next value for the question unanswered by the subject.

4. The processor implemented method as claimed in claim 2, wherein the risk weightage and the coefficient value are obtained from one or more clinical guidelines.

5. The processor implemented method as claimed in claim 1,

wherein the plurality of risk scores are computed based on a product of the coefficient value of each of the plurality of predefined questions with corresponding weight, and

wherein the plurality of risk scores are computed based on a product of, for each question, its coefficient and its corresponding weight.

6. The processor implemented method as claimed in claim 1, wherein each proportionate score corresponding to each risk score is iteratively computed by summing an incremental proportionate value for a current term to a maximum value, and sequentially computing a next term, and

$$\text{proportionate score } (\Delta p_{n+1}) = \left(\frac{S_{n+1}}{MaxValue}\right) * (MaxValue - p_n)$$

wherein the maximum value is a scale of a score list $MaxValue = \sum_{i=1}^{n} S_i$, where $s_i \in Scores_{list}$ and $n = | Scores_{list}|$.

7. The processor implemented method as claimed in claim 6, wherein the previous term $\Delta p_{n-1}$ is (i) the sum of a highest risk score, and (ii) the incremental proportionate value for the previous term which is obtained as the product of each risk score of the previous term with respect to the maximum value, and a remaining residual proportion of the maximum value.

8. The processor implemented method as claimed in claim 6,

wherein the current term $\Delta p_n$ is the incremental proportionate value for the previous term which is obtained as the product of each risk score of the current term with respect to the maximum value and the remaining residual proportion of the maximum value, and

wherein the next term $\Delta p_{n+1}$ is the incremental proportionate value for the current term which is obtained as the product of each risk score of the current term with respect to the maximum value and the remaining residual proportion of the maximum value, and the current term.

9. The processor implemented method as claimed in claim 1, wherein the ranked incremental cumulative risk (RICR) scoring technique computes the ranked incremental cumulative risk score by:

initializing the risk weightage corresponding to each of the predefined question associated with the questionnaire;

obtaining the plurality of risk scores;

determining a sorted score list by sorting the plurality of risk scores in a descending order;

determining a rank list by ranking average of the sorted score list corresponding to each risk score;

detecting a correlation between two or more predefined questions risk score using a multicollinearity metric;

computing a proportionate addition score, when two or more proportionate scores from the plurality of proportionate scores two or more risk scores are highly correlated using the maximum value in the sorted score list and the rank list; and

computing the ranked incremental cumulative risk score using the plurality of risk scores, and the proportionate addition score.

**10.** The method as claimed in claim 1, wherein the action plan includes one or more mitigations relative to the pregnancy complications based on the incremental cumulative risk score for the clinician review.

**11.** A system (100), to predict pregnancy related complications comprising:

a memory (102) storing instructions;

one or more communication interfaces (106); and

one or more hardware processors (104) coupled to the memory (102) via the one or more communication interfaces (106), wherein the one or more hardware processors (104) are configured by the instructions to:

display electronically on a clinical device, a questionnaire comprising a plurality of predefined questions to be answered by a subject, and in response receiving from the subject a plurality of answers for at least a subset of the plurality of predefined questions in the questionnaire, wherein each question is mapped to one or more risk factors;

compute a plurality of risk scores using (i) the plurality of answers received from the subject for each of the plurality of predefined questions in the questionnaire, and (ii) one or more unanswered questions, wherein a default risk score is assigned to each unanswered question;

compute by a ranked incremental cumulative risk (RICR) scoring technique, a ranked incremental cumulative risk score using the plurality of risk scores, a plurality of proportionate scores, and a proportionate addition score; and

generate a risk index of the subject using the ranked incremental cumulative risk score to predict one or more pregnancy complications, and displaying the risk index, an action plan and a risk tier on the clinical device to notify a clinician with a machine-generated alert,

wherein the risk tier indicates one or more mitigations of each risk event occurring with one or more pregnancy complications at different risk levels,

wherein the risk index summarizes an overall probability of the risk event, and

wherein the action plan is recommended based on the risk index, and the risk tier.

**12.** The system as claimed in claim 11,

wherein each question of the plurality of predefined questions is mapped to the one or more risk factors empirically through a statistical model, one or more clinical trials, and one or more expert opinions, based on a predefined medical knowledge base,

wherein each of the risk factor is associated with a risk weightage and a coefficient value, and wherein each of the risk factor indicates one or more clinical observations empirically mapped to each risk event,

wherein the coefficient value includes a previous value of the question answered by the subject, a current value of the question answered by the subject, and a next value for the question unanswered by the subject, wherein the risk weightage and the coefficient value are obtained from one or more clinical guidelines,

wherein the plurality of risk scores are computed based on a product of the coefficient value of each of the plurality of predefined questions with corresponding weight,

wherein the action plan includes one or more mitigations relative to the pregnancy complications based on the incremental cumulative risk score for the clinician review, and

wherein each proportionate score corresponding to each risk score is iteratively computed by summing an incremental proportionate value for a current term to a maximum value, and sequentially computing a next term, and

$$\text{proportionate score } (\Delta p_{n+1}) = \left(\frac{s_{n+1}}{MaxValue}\right) * (MaxValue - p_n)$$

wherein the maximum value is a scale of a score list $MaxValue = \sum_{i=1}^{n} s_i$, where $s_i \in Scores_{list}$ and $n = |Scores_{list}|$.

13. The system as claimed in claim 12, wherein the previous term $\Delta p_{n-1}$ is (i) the sum of a highest risk score, and (ii) the incremental proportionate value for the previous term which is obtained as the product of each risk score of the previous term with respect to the maximum value, and a remaining residual proportion of the maximum value.

wherein the current term $\Delta p_n$ is the incremental proportionate value for the previous term which is obtained as the product of each risk score of the current term with respect to the maximum value and the remaining residual proportion of the maximum value, and

wherein the next term $\Delta p_{n+1}$ is the incremental proportionate value for the current term which is obtained as the product of each risk score of the current term with respect to the maximum value and the remaining residual proportion of the maximum value, and the current term.

14. The system as claimed in claim 11, wherein the ranked incremental cumulative risk (RICR) scoring technique computes the ranked incremental cumulative risk score by:

initializing the risk weightage corresponding to each of the predefined question associated with the questionnaire;
obtaining the plurality of risk scores;
determining a sorted score list by sorting the plurality of risk scores in a descending order;
determining a rank list by ranking average of the sorted score list corresponding to each risk score;
detecting a correlation between two or more predefined questions risk score using a multicollinearity metric;
computing a proportionate addition score, when two or more proportionate scores are highly correlated from the plurality of proportionate scores using the maximum value in the sorted score list and the rank list; and
computing the ranked incremental cumulative risk score using the plurality of risk scores, and the proportionate addition score.

15. One or more non-transitory machine-readable information storage mediums comprising one or more instructions which when executed by one or more hardware processors cause:

displaying electronically on a clinical device, a questionnaire comprising a plurality of predefined questions to be answered by a subject and in response receiving from the subject a plurality of answers for at least a subset of the plurality of predefined questions in the questionnaire, wherein each question is mapped to one or more risk factors;
computing a plurality of risk scores using (i) the plurality of answers received from the subject for each of the plurality of predefined questions in the questionnaire, and (ii) one or more unanswered questions, wherein a default risk score is assigned to each unanswered question;
computing by a ranked incremental cumulative risk (RICR) scoring technique a ranked incremental cumulative risk score using the plurality of risk scores, a plurality of proportionate scores, and a proportionate addition score; and
generating a risk index of the subject using the ranked incremental cumulative risk score to predict one or more pregnancy complications, and displaying the risk index, an action plan and a risk tier on the clinical device to notify a clinician with a machine generated- alert,

wherein the risk tier indicates one or more mitigations of each risk event occurring with one or more pregnancy complications at different risk levels,
wherein the risk index summarizes an overall probability of the risk event, and
wherein the action plan is recommended based on the risk index, and the risk tier.

System **100**

Hardware Processor(s)
**104**

I/O Interface(s) **106**

Memory **102**

Modules **108**

**FIG.1**

200

| display electronically on a clinical device, a questionnaire comprising a plurality of predefined questions mapped to one or more risk factors to be answered by a subject, and in response receiving from the subject a plurality of answers |
|---|

202

| compute a plurality of risk scores using (i) the plurality of answers received from the subject for each of the plurality of predefined questions in the questionnaire, and (ii) one or more unanswered questions, wherein a default risk score is assigned to each unanswered question |
|---|

204

| compute by a ranked incremental cumulative risk (RICR) scoring technique, a ranked incremental cumulative risk score using the plurality of risk scores, a plurality of proportionate scores, and a proportionate addition score |
|---|

206

| generate a risk index of the subject using the ranked incremental cumulative risk score to predict one or more pregnancy complications, and displaying the risk index, an action plan and a risk tier on the clinical device. |
|---|

208

**FIG. 2**

$$\Delta p2 = (s2/MaxValue).(MaxValue-p1)$$

$$p_n = p1 + \triangle p2 + \triangle p3 + \triangle p4 + \triangle p5$$

$p_n$ = Cumulative Score after incremental proportionate addition

**FIG. 3**

**FIG.4**

Europäisches
Patentamt

European
Patent Office

Office européen
des brevets

# EUROPEAN SEARCH REPORT

**Application Number**

EP 25 22 5573

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PALLEPOGULA DINESH RAJ ET AL: "A Systematic Review of Antenatal Risk Scoring Systems in India to Predict Adverse Neonatal Outcomes", THE JOURNAL OF OBSTETRICS AND GYNECOLOGY OF INDIA SPRINGER INDIA, INDIA, vol. 72, no. 3, 12 July 2021 (2021-07-12), pages 181-191, XP037896308, ISSN: 0971-9202, DOI: 10.1007/S13224-021-01484-Z [retrieved on 2021-07-12] * abstract; tables 1-5 * * page 181, column 2, paragraph 1 - page 182, column 1, paragraph 5 * * page 183, column 1, paragraph 5 - page 190, column 1, paragraph 1 * ----- | 1-15 | INV. G16H50/30 G16H10/20 G16H50/20 |
| A | Anand Bhavna ET AL: "IMPORTANCE OF DEVELOPING A NEW MODIFIED HIGH RISK PREGNANCY SCORING SYSTEM", Indian Obstetrics and Gynaecology. 2017;7(1)., 1 April 2017 (2017-04-01), pages 10-14, XP093389375, Retrieved from the Internet: URL:https://iog.org.in/journal/index.php/iog/article/download/332/329 [retrieved on 2026-04-20] * abstract; tables 1-4 * * page 10, column 1, paragraph 1 - page 14, column 1, paragraph 1 * ----- | 1-15 | |
| A | EP 4 012 717 A1 (KONINKLIJKE PHILIPS NV [NL]) 15 June 2022 (2022-06-15) * abstract * * paragraph [0015] - paragraph [0042] * * paragraph [0047] - paragraph [0117] * ----- | 1-15 | **TECHNICAL FIELDS SEARCHED (IPC)** <br><br> G16H |

The present search report has been drawn up for all claims

| Place of search | Date of completion of the search | Examiner |
|---|---|---|
| Munich | 20 April 2026 | Menschner, Philipp |

## ANNEX TO THE EUROPEAN SEARCH REPORT
## ON EUROPEAN PATENT APPLICATION NO.

EP 25 22 5573

This annex lists the patent family members relating to the patent documents cited in the above-mentioned European search report.
The members are as contained in the European Patent Office EDP file on
The European Patent Office is in no way liable for these particulars which are merely given for the purpose of information.

20-04-2026

| Patent document cited in search report | | Publication date | Patent family member(s) | | Publication date |
|---|---|---|---|---|---|
| EP 4012717 | A1 | 15-06-2022 | CN | 114613487 A | 10-06-2022 |
| | | | EP | 4012717 A1 | 15-06-2022 |
| | | | WO | 2022122605 A1 | 16-06-2022 |

EPO FORM P0459

For more details about this annex : see Official Journal of the European Patent Office, No. 12/82

**REFERENCES CITED IN THE DESCRIPTION**

**Patent documents cited in the description**

- IN 202421103823